# EUROPEAN PATENT APPLICATION

(11) **EP 4 599 862 A1**
(43) Date of publication of application: **13.08.2025**
(21) Application number: 23906314.2
(22) Date of filing: 16.06.2023
(51) Int. Cl.: A61M 5/00, A61M 5/19, A61M 5/32

(54) **DRUG ADMINISTRATION TOOL**

(30) Priority: 23.12.2022 JP 2022206768
(71) Applicant: Terumo Kabushiki Kaisha, Tokyo 151-0072 (JP)
(72) Inventor: OGAWA, Junichi, Nakakoma-gun, Yamanashi 409-3853 (JP); NUNOME, Remi, Nakakoma-gun, Yamanashi 409-3853 (JP)
(74) Representative: KIPA AB
(86) International application number: PCT/JP2023/022475
(87) International publication number: WO 2024/134928

(57) **Abstract**

A medication administration device 1 includes a holding member 30 that bundles and holds a plurality of auto-injectors 5, the holding member 30 includes a housing holding portion 31, a cap covering portion 32, and a breakable connection portion 33, the housing holding portion 31 and the cap covering portion 32 are separated by breaking the breakable connection portion 33, and the separated housing holding portion 31 maintains a plurality of auto-injector bodies 50 in a bundled and held state, as well as the separated cap covering portion 32 is allowed to be detached from the medication administration device 1 alone or simultaneously with a plurality of the caps 56.

## Description

### Technical Field

The present invention relates to a medication administration device including a plurality of auto-injectors and a holding member that bundles and holds the plurality of auto injectors.

### Background Art

In the related art, there has been known a device (auto-injector) for automatically or semi-automatically injecting (administering) a medication (such as a medical liquid). In such an auto-injector, for example, as disclosed in Patent Literature 1 (JP 2015-533562 W), a driving mechanism (90) for delivering a certain amount of medication is released by a predetermined operation, and the medication in a medication container (24) is ejected through a delivery member (28).

Auto-injectors are standardized to some extent depending on types and applications of medications. Therefore, some patients may be required in some cases to use a plurality of auto-injectors to administer medications, according to appropriate doses of medications.

### Citation List

### Patent Literature

Patent Literature 1: JP 2015-533562 W (WO 2014 053451 A, US 2015-273162 A, EP 2903670 A)

### Summary of Invention

### Technical Problem

In a case where medications are administered using a plurality of auto-injectors, a lot of time and effort arise also in preparing for administration of medications. It takes a lot of time and effort to, for example, unpackage each of the plurality of auto-injectors, take off the caps, and furthermore, as necessary, attach the plurality of auto-injectors to an aid for simultaneous administration. In addition, the cap attached to a distal end part of the auto-injector is to restrain an injection needle or the like from being touched from the outside before use and is effective to enhance the safety of the auto-injector. Here, there is a relationship that is difficult to achieve at the same time between making it difficult for the cap to fall off in order to enhance the safety and making it easy to remove the cap in order to reduce the time and effort in preparing for administration.

Thus, an object of the present invention is to provide a medication administration device including a plurality of auto-injectors and a holding member that bundles and holds the plurality of auto-injectors, the medication administration device being capable of reducing time and effort in preparing for administration and having high safety before use.

### Solution to Problem

The above object is achieved by the following.
(1) A medication administration device including: a plurality of auto-injectors each including an auto-injector body in which a prefilled syringe and a gasket pushing mechanism are housed in a housing having a cylindrical shape, and a cap removably attached to a distal end part of the auto-injector body; and a holding member that bundles and holds the plurality of auto-injectors, in which
   the holding member includes a housing holding portion that holds the housings of the plurality of auto-injectors, a cap covering portion that covers the caps of the plurality of auto-injectors, and a breakable connection portion that separably connects the housing holding portion and the cap covering portion, and
   the housing holding portion and the cap covering portion are separated by breaking the breakable connection portion, and the separated housing holding portion maintains a plurality of the auto-injector bodies in a bundled and held state, as well as the separated cap covering portion is allowed to be detached from the medication administration device alone or simultaneously with a plurality of the caps.

In addition, the above-described embodied mode may include the following.
(2) The medication administration device according to (1) above, in which the separated cap covering portion is allowed to be detached from the medication administration device simultaneously with the plurality of the caps.
(3) The medication administration device according to (1) or (2) above, in which the holding member is a film material.
(4) The medication administration device according to (3) above, in which the film material is a heat-shrinkable film material.
(5) The medication administration device according to any one of (1) to (4) above, in which the holding member is one piece of a film-shaped packaging material, and
   the breakable connection portion is a breakable portion that has a linear or band-like shape and is provided on the holding member.
(6) The medication administration device according to (5) above, in which the breakable portion is formed with a cut or a thin portion extending intermittently and linearly, and does not pass through the holding member.
(7) The medication administration device according to (5) or (6) above, in which the breakable connection portion is a breakable portion that has a band-like shape and is provided on the holding member, and the breakable portion includes a breakage start portion extending in a width direction of the breakable portion.
(8) The medication administration device according to (7) above, in which the breakage start portion is formed with a cut or a thin portion extending intermittently and linearly in the width direction of the breakable portion.
(9) The medication administration device according to (7) or (8) above, including a gripping tab having one end fixed to the breakable portion and another end provided as a free end, in which the one end of the gripping tab is fixed at a position close to the breakage start portion.
(10) The medication administration device according to any one of (1) to (9) above, including a spacer member that is disposed between the housings of adjacent two of the auto-injectors, and maintains the auto-injector bodies of the adjacent two of the auto-injectors in an isolated state from each other.
(11) The medication administration device according to (10) above, in which the housing holding portion is a film-shaped packaging material, and
   the housing holding portion covers outer peripheries of the housings of the adjacent two of the auto-injectors between which the spacer member is disposed, and maintains the auto-injector bodies of the adjacent two of the auto-injectors in an isolated state from each other and in an aligned state side by side.
(12) The medication administration device according to any one of (1) to (11) above, in which the cap covering portion is a film-shaped packaging material, and covers outer peripheries of the caps of adjacent two of the auto-injectors, and the caps of the adjacent two of the auto-injectors abut on each other in the cap covering portion.

### Brief Description of Drawings

Fig. 1 is a front view illustrating an embodiment of a medication administration device of the present invention.
Fig. 2 is a front view illustrating a state of the medication administration device illustrated in Fig. 1 with a holding member excluded therefrom.
Fig. 3 is a cross-sectional view taken along line A-A in Fig. 1.
Fig. 4 is a cross-sectional view taken along line B-B in Fig. 1.
Fig. 5 is a front view illustrating an example of an auto-injector used in the medication administration device of the present invention.
Fig. 6 is a front view for explaining a state of the auto-injector illustrated in Fig. 5 in which administration of a medication has been completed.
Fig. 7 is an explanatory front view for explaining a method for using the medication administration device illustrated in Fig. 1.
Fig. 8 is an explanatory front view for explaining a method for using the medication administration device illustrated in Fig. 1.
Fig. 9 is an explanatory front view for explaining a method for using the medication administration device illustrated in Fig. 1.
Fig. 10 is an explanatory front view for explaining a method for using the medication administration device illustrated in Fig. 1.
Fig. 11 is a front view illustrating another embodiment of a medication administration device of the present invention.
Fig. 12 is an explanatory front view for explaining a method for using the medication administration device illustrated in Fig. 11.
Fig. 13 is a front view illustrating another embodiment of a medication administration device of the present invention.
Fig. 14 is a front view illustrating another embodiment of a medication administration device of the present invention.
Fig. 15 is a front view illustrating another embodiment of a medication administration device of the present invention.
Fig. 16 is a front view illustrating another embodiment of a medication administration device of the present invention.
Fig. 17 is a front view illustrating another embodiment of a medication administration device of the present invention.
Fig. 18 is a cross-sectional view taken along line C-C in Fig. 17.
Fig. 19 is a cross-sectional view taken along line D-D in Fig. 17.
Fig. 20 is a front view illustrating another embodiment of a medication administration device of the present invention.
Fig. 21 is a front view illustrating another embodiment of a medication administration device of the present invention.

### Description of Embodiments

A medication administration device of the present invention will be described with reference to embodiments illustrated in the drawings.

A medication administration device 1 of the present invention is a medication administration device 1 including: a plurality of (here, two) auto-injectors 5 each including an auto-injector body 50 in which a prefilled syringe 52 and a gasket pushing mechanism 53 are housed in a housing 51 having a cylindrical shape, and a cap 56 removably attached to a distal end part of the auto-injector body 50; and a holding member 30 that bundles and holds the plurality of auto-injectors 5. The holding member 30 includes a housing holding portion 31 that holds the housings 51 of the plurality of auto-injectors 5, a cap covering portion 32 that covers the caps 56 of the plurality of auto-injectors 5, and a breakable connection portion 33 that separably connects the housing holding portion 31 and the cap covering portion 32, in which the housing holding portion 31 and the cap covering portion 32 are separated by breaking the breakable connection portion 33, and the separated housing holding portion 31 maintains the plurality of auto-injector bodies 50 in a bundled and held state, as well as the separated cap covering portion 32 is allowed to be detached from the medication administration device alone or simultaneously with a plurality of the caps 56.

First, an outline of an auto-injector used in the medication administration device 1 of the present embodiment will be described herein with reference to embodiments illustrated in the drawings. Various known auto-injectors are employable as the auto-injector, and details of the auto-injector 5 will be omitted herein except for a predetermined configuration. The auto-injector 5 of the present embodiment includes the auto-injector body 50 in which the prefilled syringe 52 and the gasket pushing mechanism 53 are housed in the housing 51 having a cylindrical shape, and the cap 56 removably attached to a distal end part of the auto-injector body 50.

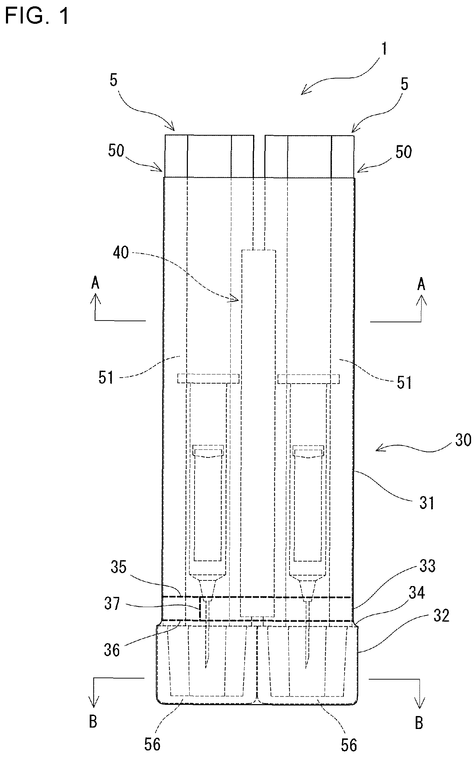

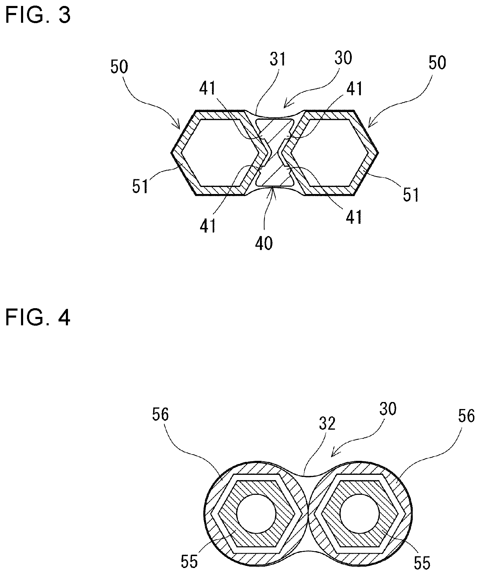

The housing 51 of the auto-injector 5 (auto-injector body 50) is a cylindrical member formed with a hard or semi-hard resin, having a substantially hexagonal cross section perpendicular to an axial direction, as illustrated in Figs. 1 and 3. Note that the shape of the cross section of the housing of the auto-injector body 50 perpendicular to the axial direction may have, for example, a circle, a rounded polygon, or a rounded quadrangle with each side curved outward, apart from the illustrated shape.

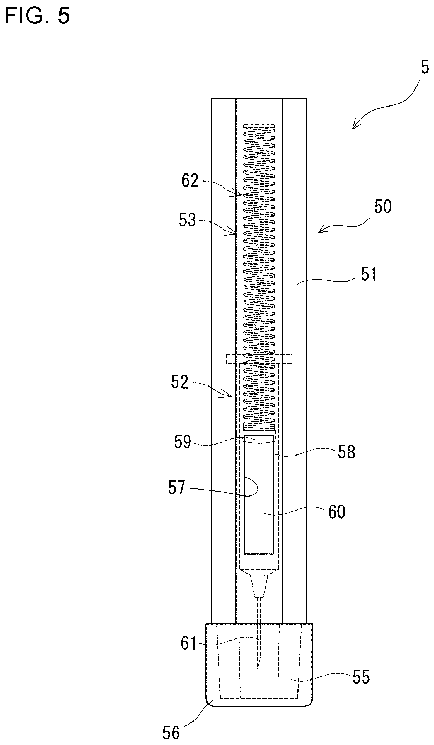

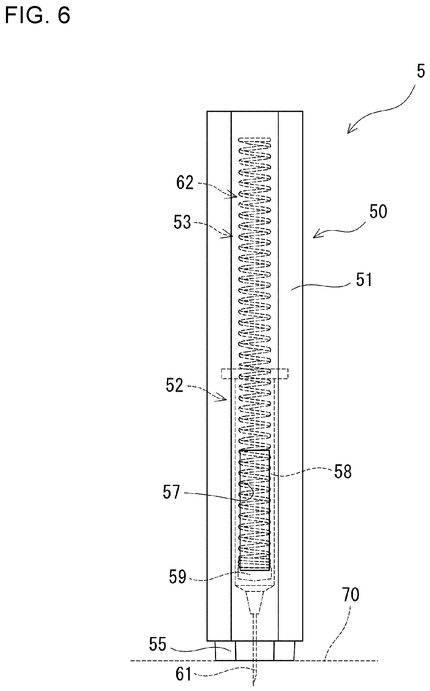

An end portion (the upper end portion in Fig. 5) of the housing 51 of the auto-injector body 50 on one side in the axial direction is closed. An end portion (the lower end portion in Fig. 5) of the housing 51 on another side in the axial direction is opened and provided with a pressing portion 55 (needle cover) protruding from the housing 51, as illustrated in Figs. 5 and 6. The pressing portion 55 is movable relative to the housing 51 within a predetermined range. As illustrated in Fig. 5, the cap 56 is removably attached to a distal end part of the auto-injector body 50 in order to restrain the pressing portion 55 and an injection needle 61 from being touched from the outside before use.

In the present embodiment, as illustrated in Fig. 5, an outer shape (outer diameter) of the cap 56 is larger than an outer shape of the distal end part of the auto-injector body 50. In other words, in the auto-injector 5, a width direction dimension (a dimension in the left-right direction in Fig. 5) of the cap 56 part is larger than a width direction dimension of the auto-injector body 50 as viewed from the front, and side portions of the cap 56 protrude outward. In this embodiment, as illustrated in Fig. 4, the cap 56 has a circular cross section and is a cylindrical member with a closed distal portion. Additionally, the entire tubular side surface of the cap 56 is larger than the entire tubular side surface of the auto-injector body 50, and the entire side portion of the cap 56 protrudes outward.

The auto-injector 5 includes a window 57 that is formed on a side wall of the housing 51 and used to visually check the inside of the prefilled syringe 52. A typical auto-injector includes a window that allows the inside of a prefilled syringe, specifically, the movement of a gasket housed inside the prefilled syringe to be visually checked. Confirming the movement of the gasket from this window allows a progress and end of administration of a medication (medical liquid) to be observed.

The window 57 of the auto-injector 5 is a substantially rectangular through-hole formed in a side wall of the housing 51. This allows the inside of the housing 51 to be visually checked from the outside of the auto-injector 5 through the window 57. The window 57 is formed in a distal side part of the auto-injector 5. With this configuration, the inside of the prefilled syringe 52 housed in the housing 51 (more specifically, the movement of a gasket 59 in the prefilled syringe 52) can be visually checked from the outside of the auto-injector 5 through the window 57. Although not illustrated, the window 57 is preferably formed on each of two facing surfaces of the side wall of the housing 51. Note that the shape, position to form, and number of windows are not limited to those illustrated above. For example, the number of windows may be one or three or more. However, from a viewpoint of visibility of the inside of the prefilled syringe through the window, it is preferable that windows are formed at least on facing surfaces (parts) of the side wall of the housing.

The prefilled syringe 52 is housed inside the housing 51. The prefilled syringe 52 includes a barrel 58, the gasket 59 slidably housed in the barrel 58 in a liquid-tight manner, and a medication (medical liquid) 60 filled in the barrel 58.

The barrel 58 is a transparent or translucent cylindrical member. A proximal end part (the upper end portion in Fig. 5) of the barrel 58 (barrel body) is provided with an outward protruding flange. A distal end part (the lower end portion in Fig. 5) of the barrel 58 is provided with a nozzle having a diameter decreasing in a distal direction, and the metal hollow injection needle 61 is attached to the nozzle. The inside and outside of the barrel 58 communicate with each other through the injection needle 61. A distal portion of the injection needle 61 has a puncture needle tip. The barrel 58 is housed (fixedly installed) inside the housing 51 so as not to move relative to the housing 51. Examples of the material to form the barrel 58 include glass and plastic.

The gasket 59 is housed in the barrel 58 (barrel body). The gasket 59 is movable, being in contact with an inner surface of the barrel 58 in a liquid-tight manner. A preferable example of the material to form the gasket 59 includes rubber having elasticity.

The inside of the prefilled syringe 52, specifically, a space defined by the inner surface of the barrel 58 and the gasket 59 inside the barrel 58 is filled with the medical liquid 60. The medical liquid 60 filled in the prefilled syringe 52 is, for example, a solution, gel, or suspension containing a medication. An employable medication is not practically limited as long as it is not a medication unsuitable for transdermal administration.

Examples of the medication contained in the medical liquid include a protein preparation, an antibody preparation, hyaluronic acid, an antibacterial drug, an antiviral drug, a vaccine, an antitumor drug, an immunosuppressant, a steroid, an anti-inflammatory drug, an antirheumatic drug, an arthritis therapeutic drug, an antihistamine, an antiallergic drug, a diabetes therapeutic drug, a hormone agent such as a growth hormone, a bone calcium metabolic drug, a vitamin, a blood preparation, a hematopoietic drug, an antithrombotic drug, an antihyperlipidemic drug, an antiarrhythmic drug, a vasodilator, a prostaglandin, a calcium antagonist, an angiotensin-converting enzyme (ACE) inhibitor, a β-blocker, an antihypertensive, a diuretic, a xanthine derivative, a β-agonist, an anti-asthmatic drug, a cough suppressant, an expectorant, an anticholinergic drug, an anti-diarrheal drug, a gastrointestinal drug, an antiulcer drug, a purgative, a sleep medication, a sedative, an antipyretic, a cold medication, an antiepileptic drug, an antipsychotic, an antidepressant, an antianxiety drug, a central nervous system stimulant, a parasympathomimetic, a sympathomimetic, an antiemetic, a central stimulant, an anti-parkinsonian drug, a muscle relaxer, an antispasmodic, an anesthetic, an antipruritic, an anti-migraine drug, an oligonucleotide, and a genetic disease drug.

The gasket pushing mechanism 53 is housed inside the housing 51. The detailed structure of the gasket pushing mechanism 53 is omitted herein, but note that, for example, the gasket pushing mechanism 53 includes a coil spring 62 capable of pushing the gasket 59, as illustrated in Figs. 5 and 6. The coil spring 62 is compressed in advance and confined so as not to exert its biasing force before use of the auto-injector 5. The gasket pushing mechanism 53 is actuated by a predetermined operation with respect to the auto-injector 5 (by changing the state of the auto-injector 5 to a predetermined state). In other words, the predetermined operation actuates the gasket pushing mechanism 53 to push (bias) the gasket 59 by the coil spring 62, thereby moving the gasket 59 inside the barrel 58 toward the distal direction of the prefilled syringe 52 (barrel 58).

The actuation of the auto-injector 5 of the present embodiment will now be described briefly. When using the auto-injector 5, first, the cap 56 is removed from the auto-injector 5. Next, the housing 51 is gripped and the pressing portion 55 is pressed against an administration site 70, and the housing 51 is advanced toward the administration site 70. Accordingly, as illustrated in Fig. 6, the pressing portion 55 moves inside the housing 51, and the injection needle 61 is exposed (protrudes) from the pressing portion 55, whereby the administration site 70 is punctured with the injection needle 61. This operation (allowing a predetermined length of the pressing portion 55 to enter the housing 51) actuates the gasket pushing mechanism 53. In a state where the administration site 70 is punctured with the injection needle 61, the movement of the gasket 59 in the distal direction of the prefilled syringe 52 (barrel 58) by the gasket pushing mechanism 53 (coil spring 62) causes ejection of the medical liquid 60 from the injection needle 61, whereby the medical liquid 60 is administered to a patient. After the administration of the medical liquid 60, the auto-injector 5 pressed against the administration site 70 is canceled and the housing 51 is retracted, whereby the pressing portion 55 returns to the state before use and the injection needle 61 is housed again in the housing 51 (pressing portion 55).

The auto-injector 5 of the present embodiment is what is called a two-step auto-injector in which the gasket pushing mechanism 53 is automatically actuated by advancing the housing 51 (by allowing a predetermined length of the pressing portion 55 to enter the housing 51) in a state where the pressing portion 55 is pressed against the administration site 70, thereby administering the medical liquid 60 to a patient. What is called a three-step auto-injector may also be employed in which a gasket pushing mechanism is actuated by a predetermined operation (for example, by pushing a separate actuation button) in a state where the administration site 70 of a patient is punctured with an injection needle.

The medication administration device 1 of the present embodiment includes a plurality of (here, two) auto-injectors 5 and includes the holding member 30 that bundles and holds the plurality of auto-injectors 5.

In the present embodiment, as illustrated in Figs. 1, 3, and 4, the holding member 30 is formed with a film material. In addition, the holding member 30 also functions as a packaging material. The holding member 30 is formed with a film material and adheres fast to the auto-injectors, which allows the outer shape of the medication administration device 1 including the holding member 30 to be miniaturized. Here, the film material is a thin member, and various known film materials can be used.

In addition, as a constituent material of the holding member 30 (film material), a material having heat shrinkability is preferably used. Examples of the material of the holding member 30 (film material) include polyesters such as polyethylene terephthalate and polybutylene terephthalate, polyolefins such as polyethylene and polypropylene, polyvinyl chloride, polystyrene, and polymer alloys containing them.

The film material may be a single-layer film material, or a laminated film material constituted by a plurality of layers having different constituent materials can also be used. Note that the film material is not particularly restrictive in terms of constituent material and thickness. The film material preferably has transparency that allows the auto-injector to be visually checked from the outside of the medication administration device.

In addition, the holding member 30 (film material) may be a stretched film obtained by stretching a film formed with the above-mentioned materials. Furthermore, the holding member 30 (film material) may be a heat-shrinkable cylindrical material formed with the above-mentioned materials.

In the present embodiment, a cylindrical material made with a heat-shrinkable film material is suitably used as the film material constituting the holding member 30. The heat-shrinkable film material is a film material having a property of being shrunk when heat is applied thereto. The heat-shrinkable film material may sometimes be referred to as a shrink film material. The heat-shrinkable film material shrinks with heat applied while wrapping an object to be packaged and adheres fast to the outer surface of the object to be packaged (covers the outer periphery of the object to be packaged).

In the present embodiment, the holding member 30 is formed with one piece of a film-shaped packaging material. In other words, in this embodiment, the holding member 30 is made with one piece of a cylindrical heat-shrinkable film material and includes a film-shaped packaging material that covers and adheres fast to outer peripheries of a plurality of (two in this embodiment) auto-injectors 5 including the cap 56 and packages the auto-injectors 5. The medication administration device 1 can be handled while a plurality of auto-injectors 5 is kept in a packaged (held) state by the holding member 30 that includes a film-shaped packaging material. It is preferable that the holding member that includes a film-shaped packaging material has transparency that allows the auto-injectors to be visually checked from the outside of the medication administration device, but for example, a display indicating details (such as medication names and the amount of medications) of the auto-injectors may be printed on a part of the holding member.

The holding member 30 includes the housing holding portion 31 that holds the housings 51 of the plurality of auto-injectors 5. Here, a part of the holding member (film-shaped packaging material) 30 that covers the outer peripheries of the housings 51 and 51 of two adjacent auto-injectors 5 and 5 constitutes the housing holding portion 31. In the present embodiment, as illustrated in Fig. 1, the housing holding portion 31 does not cover a proximal end part of the housing 51 of the auto-injector 5, but the embodiment is not limited to such a mode, and the housing holding portion may cover the proximal end part of the housing of the auto-injector.

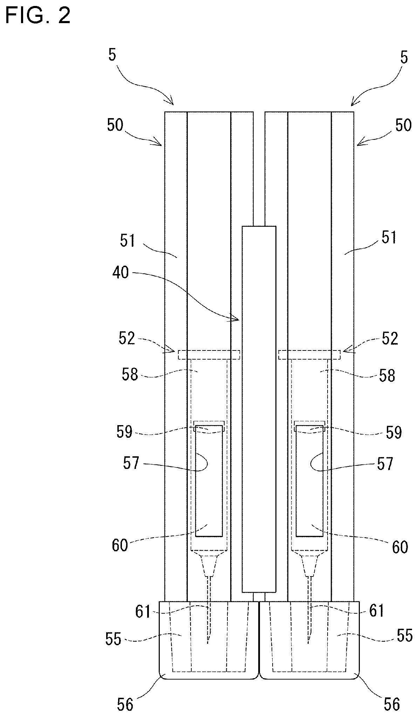

In the present embodiment, as illustrated in Figs. 1 and 2, a spacer member 40 for maintaining the auto-injector bodies 50 and 50 of two adjacent auto-injectors 5 and 5 in an isolated state from each other is disposed between the housings 51 and 51 of the two adjacent auto-injectors 5 and 5.

Specifically, the spacer member 40 has a predetermined thickness and extends by a predetermined length in the axial direction (the up-down direction in Fig. 1) of the auto-injectors 5. The spacer member 40 can maintain a predetermined interval between the two adjacent auto-injectors 5 and 5, particularly, between the injection needles 61 and 61 of the auto-injectors 5 and 5. Accordingly, the administration sites (injection sites) for medical liquids to be administered by a plurality of auto-injectors can be prevented from getting too close.

In addition, in the present embodiment, as described above, the outer shape (outer diameter) of the cap 56 is larger than the outer shape of the distal end part of the auto-injector body 50, and when the two adjacent auto-injector bodies 50 and 50 are brought into an aligned state side by side (the axes are in a parallel state), the caps 56 and 56 abut on each other, and a gap is formed between the housings 51 and 51 of the auto-injectors 5 and 5. The spacer member 40 preferably fills up this gap. In addition, the spacer member 40 preferably maintains the two adjacent auto-injectors 5 and 5 in substantially parallel while the caps 56 are in a mounted state. The spacer member 40 also preferably maintains the injection needles 61 and 61 of the two adjacent auto-injectors 5 and 5 while the caps 56 are in a detached state so as to be substantially parallel.

The spacer member 40 has a side shape corresponding to the outer shape of the housing 51 of the auto-injector 5. Here, as illustrated in Fig. 3, in the cross section perpendicular to the axial direction, the spacer member 40 has a shape with a central portion recessed to correspond to the outer shape (corner portions) of the substantially hexagonal cylindrical housings 51. Note that the side shape of the spacer member can be appropriately set in correspondence with the outer shape of the housings of the auto-injectors.

As illustrated in Fig. 3, a plurality of protrusions 41 (here, four) is formed on side portions of the spacer member 40. In the present embodiment, each of the protrusions 41 formed on a side portion of the spacer member 40 forms a rib protruding outward in a substantially semicircular cross section and extending over the entire length of the spacer member 40 in the axial direction. While the spacer member 40 is in a disposed state between the housings 51 and 51 of the two adjacent auto-injectors 5 and 5, each of the protrusions 41 abuts on an outer surface of the auto-injector 5 (housing 51). Note that the form of the protrusions formed on the side portions of the spacer member is not limited to the above-described form and may include a plurality of protrusions or the like formed substantially hemispherically, for example.

Note that the spacer member may have a configuration for holding the auto-injector (housing). For example, a housing holding portion (31f, including an accommodating portion that accommodates a part of a housing of an auto-injector so as to partially conceal the housing from the outside) to be described later can also be used as the spacer member.

In the present embodiment, the housing holding portion 31 is a film-shaped packaging material (a part of one piece of the film-shaped packaging material constituting the holding member 30), and the housing holding portion 31 covers the outer peripheries of the housings 51 and 51 of the two adjacent auto-injectors 5 and 5 between which the spacer member 40 is disposed, and maintains the auto-injector bodies 50 and 50 of the two adjacent auto-injectors 5 and 5 in an isolated state from each other and in an aligned state side by side (the axes are in a parallel state). This allows the spacer member 40 to cooperate with the holding member 30 (housing holding portion 31) and to maintain the auto-injector bodies 50 and 50 of the two adjacent auto-injectors 5 and 5 in an isolated state from each other without rattling.

The holding member 30 includes the cap covering portion 32 that covers the caps 56 of the plurality of auto-injectors 5. Here, a part of the holding member (film-shaped packaging material) 30 that covers the outer peripheries of the caps 56 and 56 of the two adjacent auto-injectors 5 and 5 serves as the cap covering portion 32. In the present embodiment, as illustrated in Fig. 1, the cap covering portion 32 covers the distal ends parts of the caps 56 (the distal portion is open), but the embodiment is not limited to such a mode, and the cap covering portion may not cover the distal ends parts of the caps, or the distal portion may not be open (closed).

In the present embodiment, the cap covering portion 32 is a film-shaped packaging material (a part of a single piece of the film-shaped packaging material constituting the holding member 30) and covers the outer peripheries of the caps 56 and 56 of the two adjacent auto-injectors 5 and 5, and the caps 56 and 56 of the two adjacent auto-injectors 5 and 5 abut on each other in the cap covering portion 32.

Specifically, in the present embodiment, as illustrated in Fig. 4, the outer shape of the cap 56 has a substantially cylindrical appearance, and outer peripheral surfaces of the two caps 56 and 56 abut on each other (are brought into close contact) linearly (linearly along the axial direction) while the auto-injector bodies 50 and 50 of the two adjacent auto-injectors 5 and 5 are in an aligned state side by side (the axes are in a parallel state). This allows the holding member 30 (cap covering portion 32) to hold (cover) the caps 56 and 56 of the two adjacent auto-injectors 5 and 5 without rattling. In addition, as will be described later, the separated cap covering portion 32 is easily detached from the medication administration device 1 simultaneously with the plurality of caps 56.

In the present embodiment, as described above, the outer shape (outer diameter) of the cap 56 is larger than the outer shape of the distal end part of the auto-injector body 50, and in the holding member 30, as illustrated in Fig. 1, a throttle portion 34 is formed on an upper portion of the cap covering portion 32. More specifically, in the holding member 30 that is made with one piece of the cylindrical heat-shrinkable film material and covers and adheres fast to the outer peripheries of the auto-injectors 5, the throttle portion 34 whose outer shape decreases upward is formed on the upper portion of the cap covering portion 32. Accordingly, the cap covering portion 32 more firmly covers the caps 56. In addition, as will be described later, the separated cap covering portion 32 is easily detached from the medication administration device 1 simultaneously with the plurality of caps 56.

The holding member 30 includes a breakable connection portion 33 that separably connects the housing holding portion 31 and the cap covering portion 32. Here, the holding member 30 is one (in other words, a single or unitary) piece of the film-shaped packaging material, and the breakable connection portion 33 is a breakable area that has a linear or band-like shape and is provided on the holding member 30. In other words, the upper portion of the holding member 30 serves as the housing holding portion 31 and the lower portion thereof serves as the cap covering portion 32 with the breakable connection portion (breakable portion) 33 as a boundary.

In the present embodiment, the breakable connection portion (breakable portion) 33 is provided over the entire circumference of the holding member 30. The breakable portion 33 includes a first breakable linear portion 35 extending over a substantially entire length (entire circumference) of the breakable portion 33, and a second breakable linear portion 36 confronting (in this embodiment, substantially parallel to) the first breakable linear portion 35 and extending over a substantially entire length (entire circumference) of the breakable portion 33. In other words, the first breakable linear portion 35 and the second breakable linear portion 36 extending to confront each other (substantially parallel in this embodiment) form the band-shaped breakable portion 33 in the holding member 30.

In the present embodiment, as illustrated in Fig. 1, the second breakable linear portion 36 is located above the proximal ends of the caps 56 (the distal ends parts of the housings 51) in the axial direction of the auto-injectors 5. Accordingly, the above-described throttle portion 34 is formed on the upper portion of the cap covering portion 32. In addition, the first breakable linear portion 35 is located above the second breakable linear portion 36 but below a lower end of the window 57 formed in the auto-injector body 50.

The breakable portion 33 is preferably formed with cuts or thin portions extending intermittently and linearly, and does not pass through the holding member 30. In the present embodiment, the first breakable linear portion 35 and the second breakable linear portion 36 are cuts (so-called half-cut portions) formed in the holding member 30 so as to extend intermittently and linearly and not to pass through the holding member 30, and have lower strength (easier to break) than other parts of the holding member 30 (parts where the first breakable linear portion 35 and the second breakable linear portion 36 are not formed).

Since the breakable linear portions 35 and 36 do not pass through the holding member (film-shaped packaging material) 30, it is possible to prevent the breakable connection portion (breakable portion) 33 from being unintentionally broken (the housing holding portion 31 and the cap covering portion 32 from being separated) before use of the medication administration device 1, to make it difficult for the cap 56 to fall off from the auto-injector 5, and to enhance the safety. Furthermore, foreign matter can be prevented from getting in the medication administration device 1 from the breakable connection portion (breakable portion) 33.

Note that, in the above-described embodiment, the breakable portion (the first breakable linear portion and the second breakable linear portion) is formed with cuts extending intermittently and linearly, but the embodiment is not limited thereto. The breakable portion (breakable linear portions) may be formed with, for example, perforations, a fold, intermittently formed dots, a continuously formed thin portion, or the like.

As described above, the breakable connection portion 33 of the present embodiment includes the band-shaped breakable portion 33 provided on the holding member 30, and the breakable portion 33 includes a breakage start portion 37 extending in the width direction of the breakable portion 33. Then, the breakage start portion 37 is preferably formed with cuts or thin portions extending intermittently and linearly in the width direction of the breakable portion 33.

Specifically, as illustrated in Fig. 1, the breakage start portion 37 of the holding member 30 is formed with cuts extending intermittently and linearly in the width direction (the up-down direction in Fig. 1) of the breakable portion 33. In addition, as illustrated in Figs. 1 and 3, the breakage start portion 37 is provided at a part of the holding member 30 (breakable portion 33) adhering fast to the auto-injector body 50 (housing 51). This makes it easy to break the breakage start portion 37 (to start breaking the breakable portion 33) from the outside of the medication administration device 1. Note that the breakage start portion may be formed at a part of the holding member not in contact with the auto-injector (and the spacer member) between two adjacent auto-injectors.

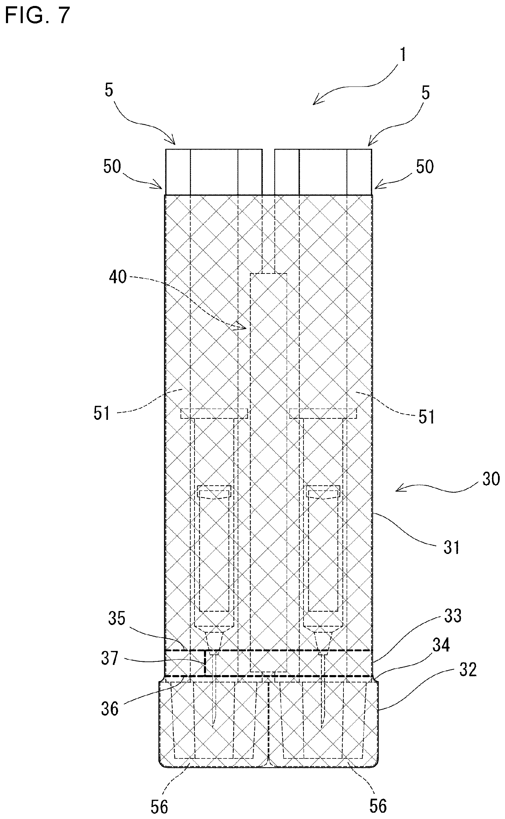

An operation when using the medication administration device 1 as described above will be described with reference to Figs. 7 to 10. Note that, in Figs. 7 to 10, for the sake of explanation, a part where the holding member (film-shaped packaging material) exists is cross-hatched.

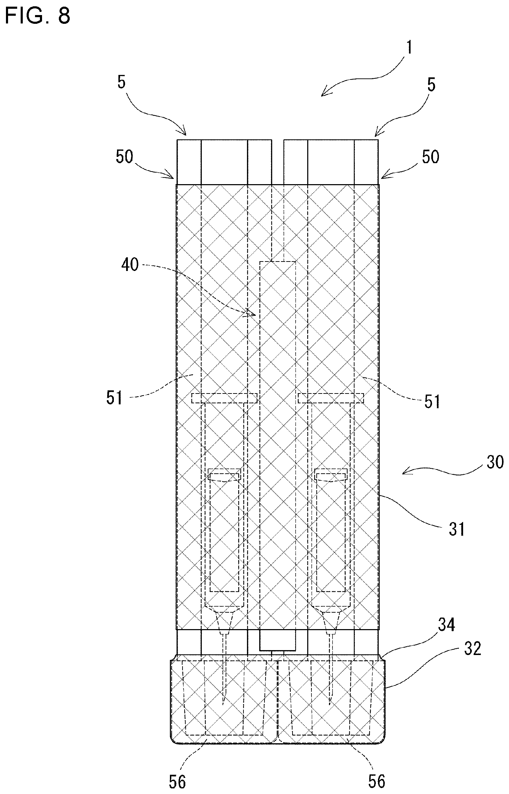

When the medication administration device 1 is used, first, the breakable connection portion (breakable portion) 33 of the medication administration device 1 illustrated in Fig. 7 is broken. Specifically, breaking of the breakable connection portion (breakable portion) 33 is started from the breakage start portion 37, the breakable connection portion (breakable portion) 33 is broken along the first and second breakable linear portions 35 and 36, and the breakable connection portion (breakable portion) 33 of the holding member 30 is moved away from the medication administration device 1. Accordingly, as illustrated in Fig. 8, the housing holding portion 31 and the cap covering portion 32 are separated.

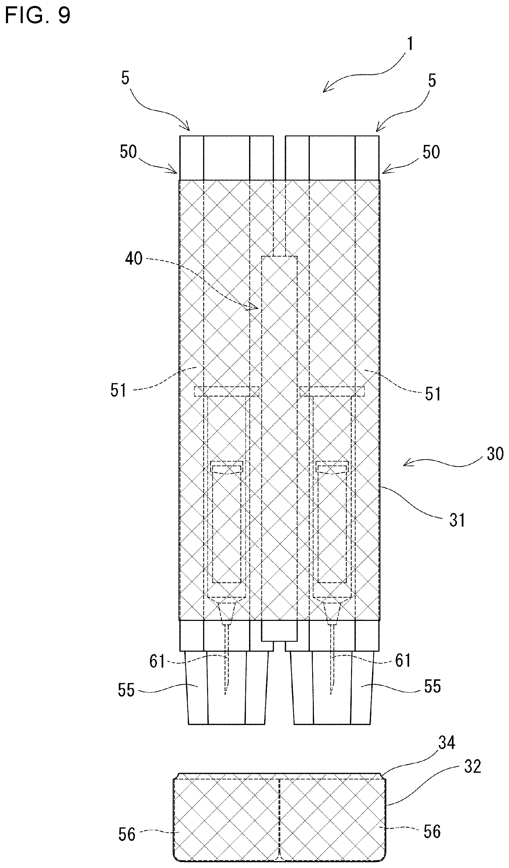

Next, as illustrated in Fig. 9, the separated cap covering portion 32 is detached from the medication administration device 1 simultaneously with the plurality of caps 56. Here, as described above, the second breakable linear portion 36 of the medication administration device 1 is located above the proximal ends of the caps 56 (the distal ends parts of the housings 51), and the throttle portion 34 is formed on the upper portion of the cap covering portion 32. Accordingly, the separated cap covering portion 32 is avoided from being unintentionally detached alone from the medication administration device 1, and furthermore, the separated cap covering portion 32 is easily detached from the medication administration device 1 simultaneously with the plurality of caps 56.

As illustrated in Fig. 9, while the cap covering portion 32 (and the caps 56) is in a detached state, the separated housing holding portion 31 maintains the plurality of auto-injector bodies 50 in a bundled and held state. In the present embodiment, the spacer member 40 is disposed between the housings 51 and 51 of the two adjacent auto-injectors 5 and 5, and the housing holding portion 31 covers the outer peripheries of the housings 51 and 51 of the two adjacent auto-injectors 5 and 5 between which the spacer member 40 is disposed, and maintains the auto-injector bodies 50 and 50 of the two adjacent auto-injectors 5 and 5 in an isolated state from each other and in an aligned state side by side (the axes are in a parallel state). This maintains the administration sites (injection sites) for the medications (medical liquids) administered by the plurality of auto-injectors in a substantially parallel state and makes it possible to prevent the administration sites (injection sites) from getting too close.

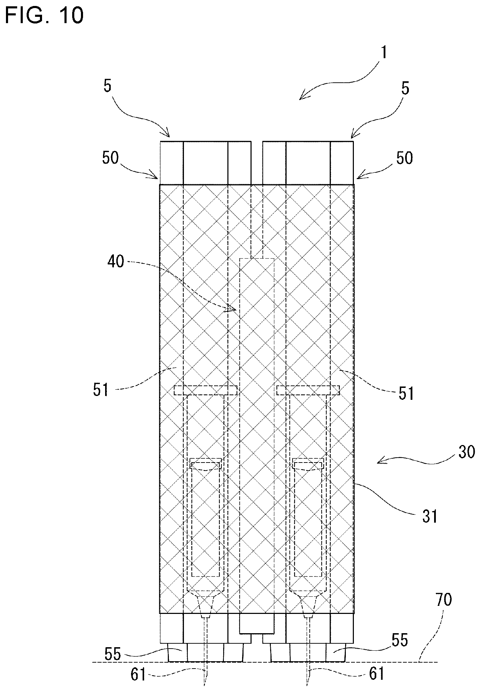

Then, as illustrated in Fig. 10, while the plurality of auto-injectors 5 is kept in a held state by the housing holding portion 31, the pressing portion 55 is pressed against the administration sites 70, and the housing 51 is further advanced toward the administration sites 70, whereby the administration of the medications (medical liquids) 60 is completed.

Note that, in the present embodiment, the breakable portion (breakable connection portion) 33 has a band-like shape, and the breakable portion 33 once broken is not restorable. This ensures that, even in a case where the cap 56 is attached to the auto-injector body 50 again after use of the medication administration device 1 (after administration of the medical liquids), it can be visually confirmed that the medication administration device 1 has been unwrapped.

Hereinafter, other embodied examples of the medication administration device of the present invention will be described with reference to the drawings. Note that, in the following description, components substantially similar to those of the medication administration device 1 described above will be referred to using corresponding names and reference signs.

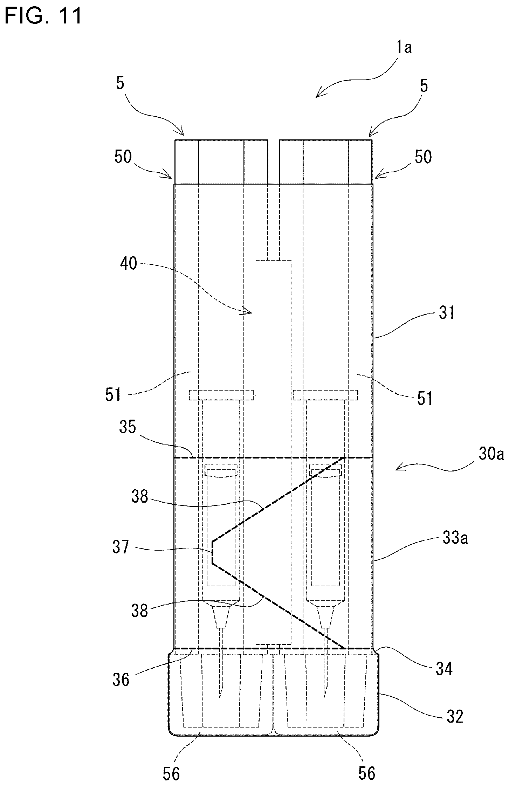

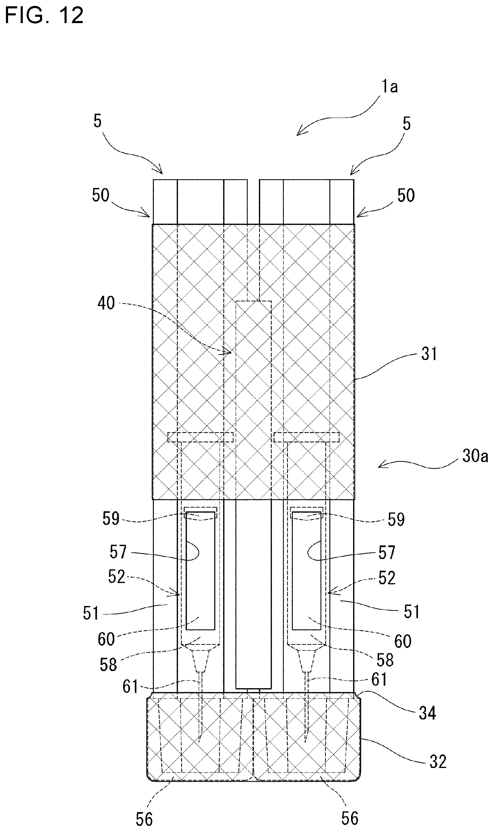

In a medication administration device 1a illustrated in Figs. 11 and 12, a first breakable linear portion 35 of a breakable connection portion (breakable portion) 33a of a holding member 30a is formed above a second breakable linear portion 36 and above upper ends of windows 57 formed in auto-injector bodies 50. Note that, in the breakable connection portion (breakable portion) 33a, breakage guide portions 38 and 38 are formed from a breakage start portion 37 toward the first breakable linear portion 35 and the second breakable linear portion 36, respectively. It is preferable that the breakage guide portions 38 are formed with cuts or thin portions extending intermittently and linearly and does not pass through the holding member 30a.

In the medication administration device 1a, the windows 57 of the auto-injectors 5 are exposed after the breakable connection portion (breakable portion) 33a is broken and moved away from the medication administration device 1a, as illustrated in Fig. 12. (Note that, in Fig. 12, for the sake of explanation, a part where the holding member (film-shaped packaging material) exists is cross-hatched.) Accordingly, even when the holding member (film-shaped packaging material) 30a has low translucency, or even when print has been made on a part of the holding member (film-shaped packaging material) 30a corresponding to the window 57, the inside of a prefilled syringe 52 can be visually checked through the window 57 at the time of using the medication administration device 1a (after eliminating the breakable connection portion 33a). In addition, according to such a mode, in a case where the medication to be filled in the prefilled syringe 52 is weak against light, the holding member (film-shaped packaging material) 30a having low translucency (having a light shielding property) can be employed.

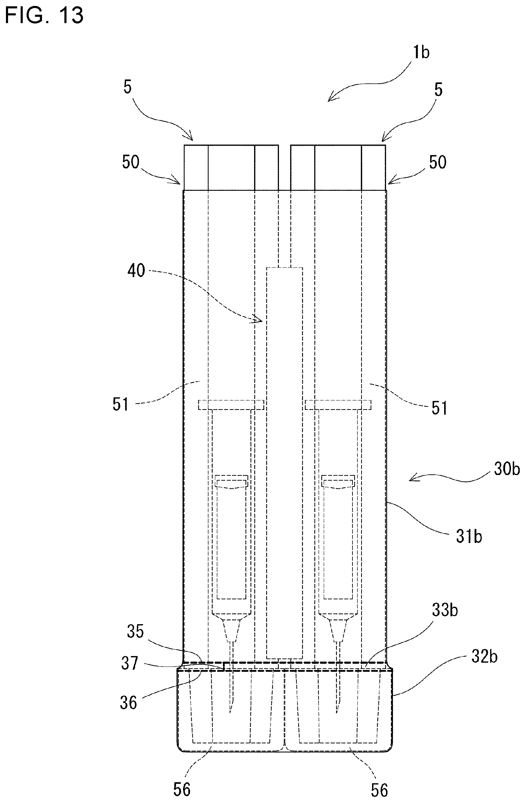

In a medication administration device 1b illustrated in Fig. 13, a second breakable linear portion 36 of a breakable connection portion (breakable portion) 33b of a holding member 30b is formed below a first breakable linear portion 35 and below upper ends of caps 56. This makes it easy, when a cap covering portion 32b does not include a throttle portion (34) as described above, to detach the separated cap covering portion 32b alone from the medication administration device 1b while a plurality of auto-injector bodies 50 is kept in a bundled and held state by the separated housing holding portion 31b.

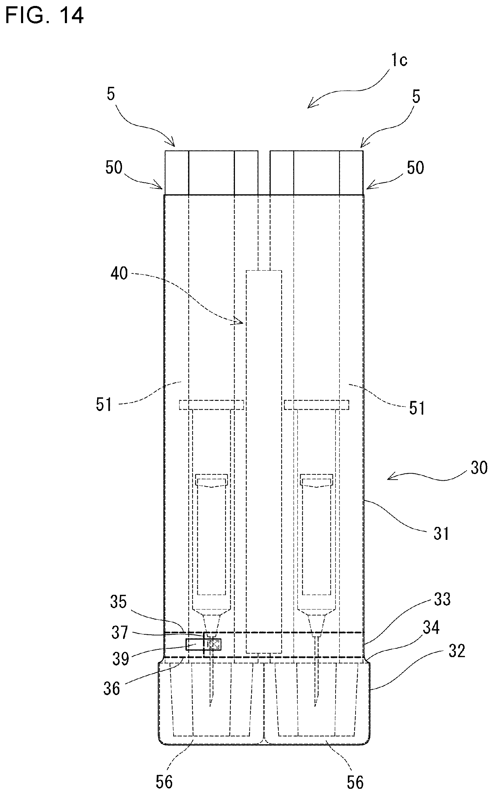

A medication administration device 1c illustrated in Fig. 14 includes a gripping tab 39 having one end fixed to a breakable portion (breakable connection portion) 33 and another end provided as a free end, and the one end of the gripping tab 39 is fixed at a position close to a breakage start portion 37 (the cross-hatched portion in Fig. 14). This allows the gripping tab 39 to be gripped and pulled when the breakable portion (breakable connection portion) 33 is broken and breaking from the breakage start portion 37 to be easily carried out.

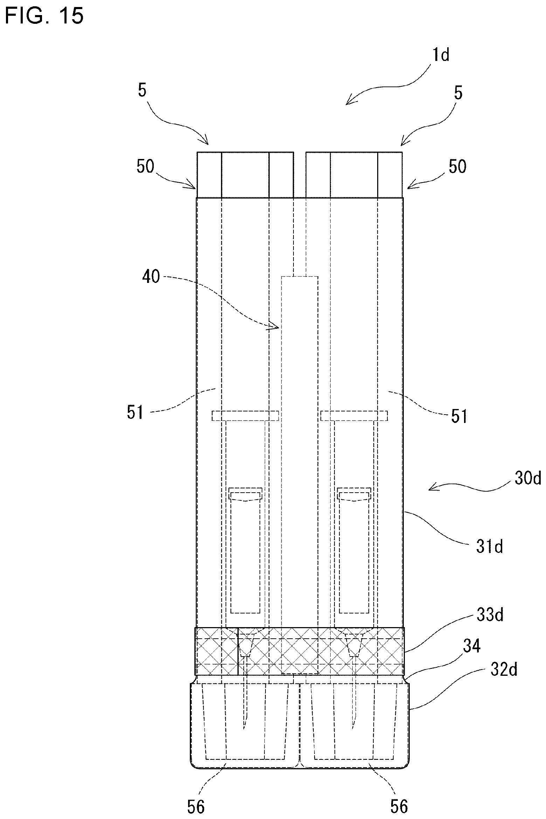

In a medication administration device 1d illustrated in Fig. 15, a breakable connection portion 33d of a holding member 30d is made with a breakable (peelable) tape member 33d (cross-hatched in Fig. 15). Specifically, a housing holding portion 31d and a cap covering portion 32d are made with separate pieces of film-shaped packaging material, and a lower end of the housing holding portion 31d and an upper end of the cap covering portion 32d are away from each other in the axial direction. The tape member 33d connects the housing holding portion 31d and the cap covering portion 32d by covering (peelably sticking) a lower end part of the housing holding portion 31d with an upper end (upper edge) part and covering (peelably sticking) an upper end part of the cap covering portion 32d with a lower end (lower edge) part. When the medication administration device 1d is used, the housing holding portion 31d and the cap covering portion 32d can be separated by peeling (breaking) the tape member 33d.

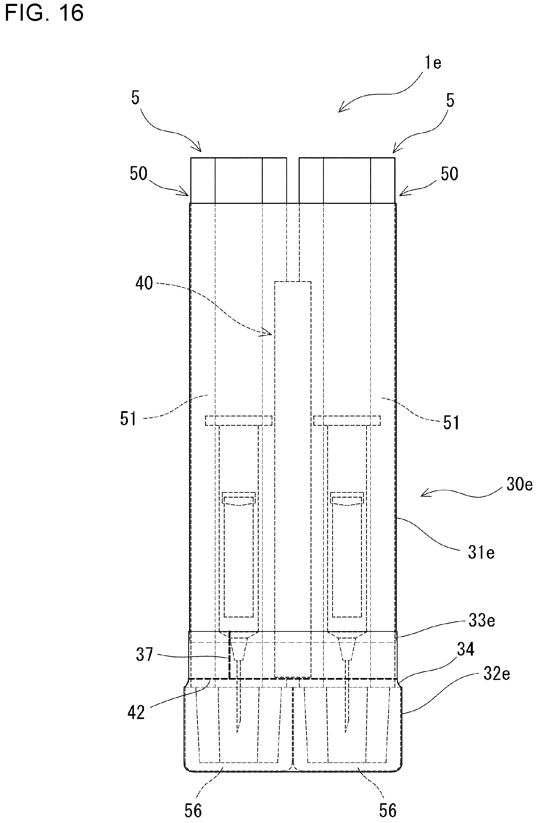

In a medication administration device 1e illustrated in Fig. 16, a breakable connection portion 33e of a holding member 30e is made with the same piece of film-shaped packaging material as a cap covering portion 32e and has an upper end part overlapping a lower end part of a housing holding portion 31e. The breakable connection portion (breakable portion) 33e includes a breakable linear portion 42 formed below a lower end of the housing holding portion 31e, and by breaking the breakable connection portion (breakable portion) 33e, the housing holding portion 31e and the cap covering portion 32e can be separated. Note that, conversely to the present embodiment, the breakable connection portion of the holding member may be made with the same piece of film-shaped packaging material as the housing holding portion and have a lower end part overlapping an upper end part of the cap covering portion.

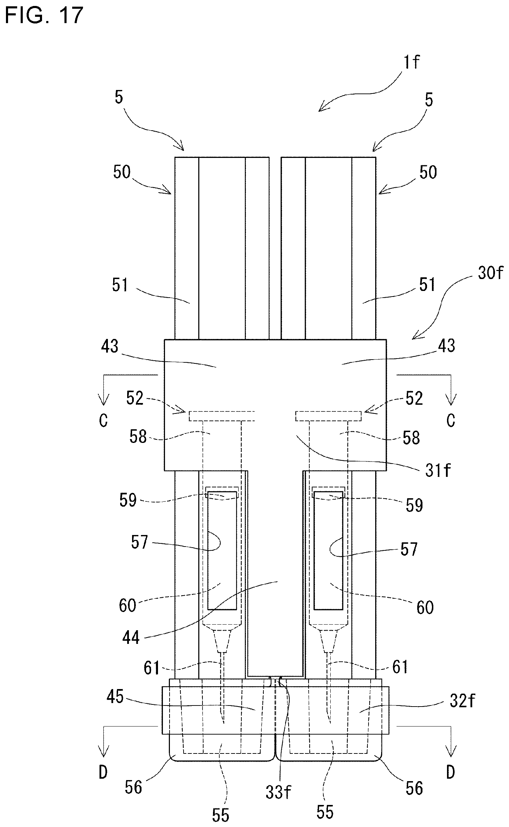

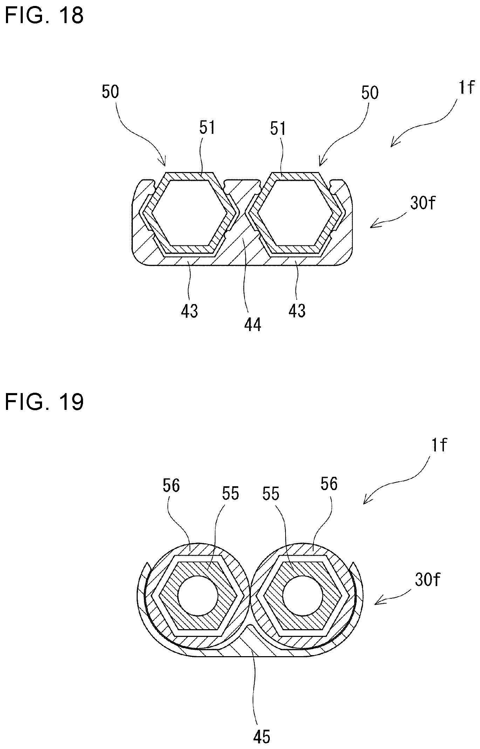

In a medication administration device 1f illustrated in Figs. 17 to 19, a holding member 30f is made with a hard or semi-hard resin. Specifically, a housing holding portion 31f includes a housing accommodating portion 43 that is made from a hard or semi-hard resin and accommodates (holds) a part of a housing 51 of an auto-injector 5 so as to partially conceal the housing from the outside. As illustrated in Fig. 18, the housing accommodating portion 43 has a substantially U-shaped cross section and extends in the axial direction (the up-down direction in Fig. 17) by a predetermined length. Preferably, the housing accommodating portion 43 firmly holds the housing 51 of the auto-injector 5 by an elastic force with an outer wall portion in an elastically deformed state.

In addition, as illustrated in Figs. 17 and 18, the housing holding portion 31f includes a partition wall portion 44 for maintaining auto-injector bodies 50 and 50 of the auto-injectors 5 and 5 accommodated (held) in the two adjacent housing accommodating portions 43 and 43 in an isolated state from each other. The partition wall portion 44 projects and extends below the housing accommodating portions 43 in the axial direction. In other words, the housing holding portion 31f of the present embodiment has both the function (housing accommodating portion) of the housing holding portion that holds the housings of the plurality of auto-injectors and the function (partition wall portion) of the spacer member that maintains the auto-injector bodies of the two adjacent auto-injectors in an isolated state from each other.

A cap covering portion 32f of the holding member 30f includes a cap accommodating portion 45 that is made from a hard or semi-hard resin and accommodates (covers) a part of caps 56 of the auto-injectors 5 so as to partially conceal the caps from the outside. As illustrated in Fig. 19, the cap accommodating portion 45 accommodates the caps 56 and 56 of the two adjacent auto-injectors 5 and 5 in an abutting state on each other in the cap accommodating portion 45. Preferably, the cap accommodating portion 45 firmly holds the caps 56 and 56 of the auto-injectors 5 and 5 by an elastic force with an outer wall portion in an elastically deformed state.

A breakable connection portion 33f of the holding member 30f has a substantially thin rod-like shape that connects the housing holding portion 31f and the cap covering portion 32f. When the medication administration device 1f is used, the breakable connection portion 33f can be broken (cut), and the separated cap covering portion 32f can be detached simultaneously with the plurality of caps 56 or only the separated cap covering portion 32f can be detached from the medication administration device 1f. In addition, by breaking the breakable connection portion 33f while the cap covering portion 32f (and the caps 56) is in a gripped state, the breaking operation for the breakable connection portion 33f and the detachment operation for the cap covering portion 32f (and the caps 56) can also be simultaneously performed.

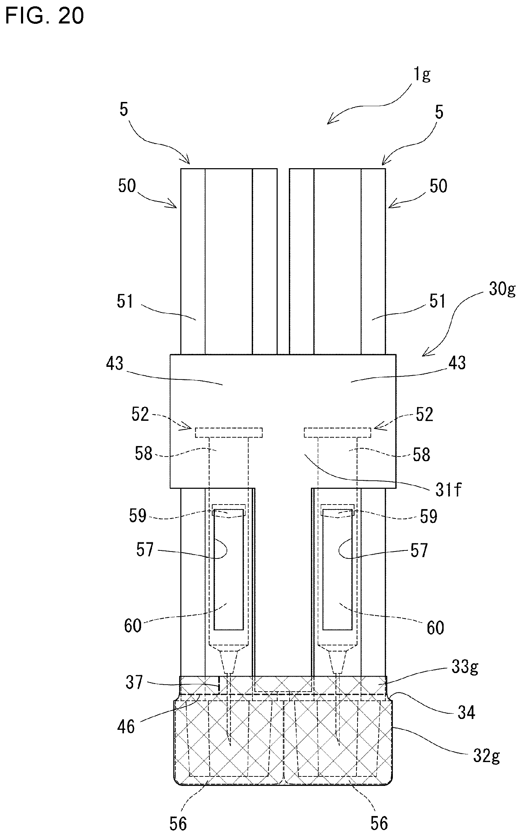

In a medication administration device 1g illustrated in Fig. 20, in a holding member 30g, the housing holding portion is the above-described resin housing holding portion 31f, while a cap covering portion 32g and a breakable connection portion 33g are made with a film material (film-shaped packaging material, cross-hatched in Fig. 20). The breakable connection portion 33g overlaps a lower end part of the housing holding portion 31f (partition wall portion 44) at an upper end part. The breakable connection portion (breakable portion) 33g includes a breakable linear portion 46 formed below a lower end of the housing holding portion 31f, and by breaking the breakable connection portion (breakable portion) 33g, the housing holding portion 31f and the cap covering portion 32g can be separated. Note that, conversely to the present embodiment, the cap covering portion of the holding member may be made from resin, and the housing holding portion and the breakable connection portion may be a film material.

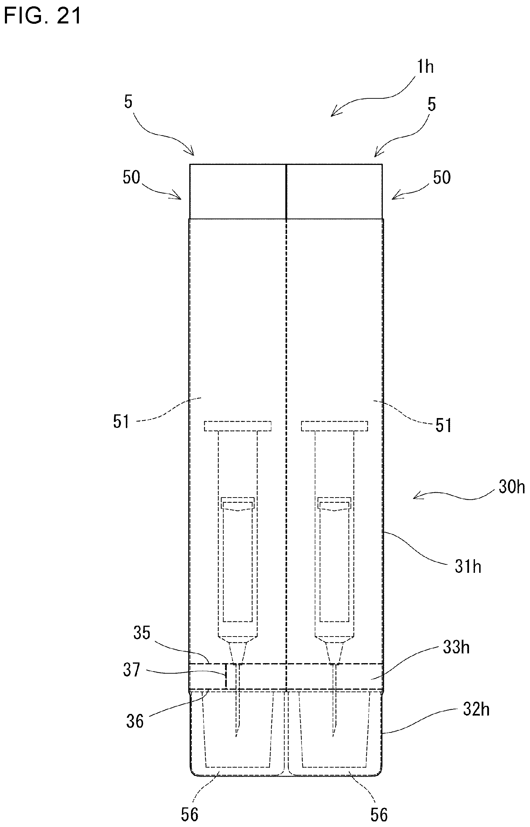

A medication administration device 1h illustrated in Fig. 21 does not include a spacer member (40). In addition, the outer shape (outer diameter) of a cap 56 is smaller than the outer shape of a distal end part of an auto-injector body 50, and the caps 56 and 56 do not abut on each other even when the auto-injector bodies 50 and 50 of the adjacent auto-injectors 5 and 5 are in an aligned state side by side (the axes are in a parallel state). In addition, in a holding member 30h, a throttle portion (34) as described above is not formed in a cap covering portion 32h. Accordingly, by breaking a breakable connection portion 33h, the separated cap covering portion 32h is easily detached from the medication administration device 1h alone while the plurality of auto-injector bodies 50 are maintained in a bundled and held state by a separated housing holding portions 31h.

Note that a width of the band-shaped breakable portion of the holding member made with a film material is preferably 1 to 20 mm, more preferably 2 to 15 mm, and especially preferably 3 to 10 mm. In addition, the breakable portion (breakable connection portion) may have a width but a narrow width (specifically, 2 mm or less) and may be practically linear.

A medication administration device of the present invention includes a holding member that bundles and holds a plurality of auto-injectors, in which the holding member includes a housing holding portion that holds housings of the plurality of auto-injectors, a cap covering portion that covers caps of the plurality of auto-injectors, and a breakable connection portion that separably connects the housing holding portion and the cap covering portion, the housing holding portion and the cap covering portion are separated by breaking the breakable connection portion, and the separated housing holding portion maintains the plurality of auto-injector bodies in a bundled and held state, as well as the separated cap covering portion is allowed to be detached from the medication administration device alone or simultaneously with a plurality of the caps.

This can make it difficult for the caps to fall off from the auto-injectors before use (while the housing holding portion and the cap covering portion are in a connected state with the breakable connection portion) and enhance the safety. In addition, at the time of use, by breaking the breakable connection portion, the plurality of caps and/or the separated cap covering portion can be detached from the medication administration device while the plurality of auto-injector bodies is maintained in a bundled and held state, and thus, time and effort in preparing for administration can be reduced.

## Claims

1. A medication administration device comprising: a plurality of auto-injectors each comprising an auto-injector body in which a prefilled syringe and a gasket pushing mechanism are housed in a housing having a cylindrical shape, and a cap removably attached to a distal end part of the auto-injector body; and a holding member that bundles and holds the plurality of auto-injectors, wherein
the holding member comprises a housing holding portion that holds the housings of the plurality of auto-injectors, a cap covering portion that covers the caps of the plurality of auto-injectors, and a breakable connection portion that separably connects the housing holding portion and the cap covering portion, and
the housing holding portion and the cap covering portion are separated by breaking the breakable connection portion, and the separated housing holding portion maintains a plurality of the auto-injector bodies in a bundled and held state, as well as the separated cap covering portion is allowed to be detached from the medication administration device alone or simultaneously with a plurality of the caps.

2. The medication administration device according to claim 1, wherein the separated cap covering portion is allowed to be detached from the medication administration device simultaneously with the plurality of the caps.

3. The medication administration device according to claim 1, wherein the holding member is a film material.

4. The medication administration device according to claim 3, wherein the film material is a heat-shrinkable film material.

5. The medication administration device according to claim 1, wherein the holding member is one piece of a film-shaped packaging material, and
the breakable connection portion is a breakable portion that has a linear or band-like shape and is provided on the holding member.

6. The medication administration device according to claim 5, wherein the breakable portion is formed with a cut or a thin portion extending intermittently and linearly, and does not pass through the holding member.

7. The medication administration device according to claim 5, wherein the breakable connection portion is a breakable portion that has a band-like shape and is provided on the holding member, and the breakable portion comprises a breakage start portion extending in a width direction of the breakable portion.

8. The medication administration device according to claim 7, wherein the breakage start portion is formed with a cut or a thin portion extending intermittently and linearly in the width direction of the breakable portion.

9. The medication administration device according to claim 7, comprising a gripping tab having one end fixed to the breakable portion and another end provided as a free end, wherein the one end of the gripping tab is fixed at a position close to the breakage start portion.

10. The medication administration device according to claim 1, comprising a spacer member that is disposed between the housings of adjacent two of the auto-injectors, and maintains the auto-injector bodies of the adjacent two of the auto-injectors in an isolated state from each other.

11. The medication administration device according to claim 10, wherein the housing holding portion is a film-shaped packaging material, and
the housing holding portion covers outer peripheries of the housings of the adjacent two of the auto-injectors between which the spacer member is disposed, and maintains the auto-injector bodies of the adjacent two of the auto-injectors in an isolated state from each other and in an aligned state side by side.

12. The medication administration device according to claim 1, wherein the cap covering portion is a film-shaped packaging material, and covers outer peripheries of the caps of adjacent two of the auto-injectors, and the caps of the adjacent two of the auto-injectors abut on each other in the cap covering portion.
